# EUROPEAN PATENT SPECIFICATION

(11) **EP 0 548 065 B1**
(45) Date of publication and mention of the grant of the patent: **27.12.1995**
(21) Application number: 89911430.0
(22) Date of filing: 01.10.1989
(51) Int. Cl.: A61M 1/16

(54) **CYLINDRICAL BLOOD HEATER/OXYGENATOR**
ZYLINDRISCHER BLUTWÄRMER UND BLUTOXYGENATOR
SYSTEME CYLINDRIQUE POUR CHAUFFER ET OXYGENER LE SANG

(43) Date of publication of application: 30.06.1993
(73) Proprietor: Minntech Corporation, Minneapolis Minnesota 55441 (US)
(72) Inventor: COSENTINO, Louis, C., Plymouth, MN 55447 (US); LEE, Jeffrey, A., Plymouth, MN 55447 (US); BAKER, Daniel, A., Minnetonka, MN 55343 (US)
(74) Representative: Rees, David Christopher
(86) International application number: US8904314
(87) International publication number: WO9104758

(56) References cited:
- EP-A- 0 114 732
- EP-A- 0 187 708
- EP-A- 0 217 759
- DE-A- 1 963 319
- US-A- 3 989 626
- US-A- 4 620 965
- US-A- 4 639 353
- US-A- 4 698 207
- US-A- 4 715 953
- US-A- 4 808 378
- US-A- 4 818 490

## Description

This invention relates to a blood oxygenator having an integral heat exchanging unit, the oxygenator being of the outside perfusion type.

In known blood oxygenators, hollow fibers are used as a means to bring blood into contact with oxygen and provide a means for removal of carbon dioxide from the blood. The fibers are typically made of a homogeneous membrane of gas-permeable material such as silicone or of hollow fibers made of a microporous membrane of hydrophobic polymeric material such as polyolefins.

There are two types of hollow fiber blood oxygenators: the inside perfusion type in which blood is passed through the bores of the hollow fibers while oxygen is passed on the outside of the hollow fibers, and the outside perfusion type. Blood oxygenators of the outside perfusion type pass oxygen through the bores of the hollow fibers while blood is flowed past the outside of the hollow fibers.

Examples of inside perfusion type hollow-fiber oxygenators are disclosed in U.S. patents 4,239,729 and 4,749,551.

In blood oxygenators of the outside perfusion type the oxygen can be distributed uniformly through the spaces between adjacent fibers and the blood can be expected to move with better mixing. However, outside perfusion has had the disadvantage of being subject to less than the desired oxygenation of the blood because of regional channeling of the blood as it passes the outsides of the hollow fibers. Blood-side convective mixing is essential for efficient gas transfer in blood oxygenators. Without such mixing, sharply defined boundary layers of fully oxygenated blood develop near the exchange surfaces and the fluxes of oxygen and carbon dioxide tend to be low. Low transport efficiency results in bulky devices with undesirable high blood priming volumes.

Outside perfusion type blood oxygenators are known in which the hollow fibers are in perpendicular orientation to the direction of blood flow so as to produce more mixing of the blood as the blood flows than inside perfusion constructions. This arrangement can bring about an improvement in oxygenation rate. However, if the number of fibers used in such a blood oxygenator is large (as is desirable) and/or the flow rate of blood is increased in order to treat large volumes of blood, problems arise. For example, unacceptable pressure drop of the blood between inlet and outlets and/or channeling of the blood between groups of fibers may occur. By channeling it is to be understood that a significant flow of blood takes place through relatively large area voids between fibers so that there is little or no mixing. As the rate of oxygen transfer primarily takes place in a thin boundary layer adjacent the hollow fibers, the effectiveness of desired oxygenation is reduced.

Examples of blood oxygenators of the outside perfusion type are disclosed in copending application PCT/US89/00146 filed January 13, 1989; WO 89/00864; and U.S. patents 3,794,468; 4,352,736; 4,622,206; 4,659,549; 4,639,353; 4,620,965; 4,791,054; and 4,808,378.

In the prior art it has been recognized that there is considerable heat loss in all extracorporeal circuits and various devices have been introduced for the purpose of maintaining the temperature of blood within the normal physiological range. Devices which combine the function of blood heating and oxygenation are known. U.S. patent 4,111,659 describes an embossed film membrane heater/oxygenator. U.S. 4,138,288 describes a bubble-type oxygenator with an integral heater at the blood outlet side of the oxygenator. U.S. 4,620,965 describes an outside perfusion type hollow fiber blood oxygenator with an associated heat exchanger, also located on the blood outlet side of the device, in which the blood flows longitudinally through the oxygenator portion of the device and generally parallel to the hollow gas exchange fibers. U.S. patents 4,639,353, 4,659,549 and 4,791,054 also disclose outside perfusion type hollow-fiber oxygenators in which blood flowing longitudinally through a generally rectangular or cylindrical device passes through multiple hollow fiber exchange chambers separated by narrow channel baffles. In some embodiments of the latter devices, separate heat and oxygen exchange chambers are provided.

U.S. 4,645,645 describes a hollow-fiber blood oxygenator to which a helical heat exchanger may be attached. Heat exchange is accomplished by passing blood across the outside of a helical coated metal coil.

U.S. 4,424,190 describes another form of hollow-fiber oxygenator with an attached heater compartment displaced longitudinally on a generally cylindrical device.

A problem with prior blood oxygenator/heater combination devices which has been recognized in the prior art is the considerable bulk, with consequent large priming volume of the combined devices. A flat device is described in WO 89/00864 and co-pending application, PCT/US89/00146 filed January 13, 1989, which locates heated exchange fibers and gas exchange fibers in adjacent compartments separated by a porous wall so as to eliminate collection and distribution manifolds between the devices. Such flat devices, however, are difficult to manufacture because of the difficulty of properly packing the gas exchange fibers for optimal efficiency.

According to one aspect of the present invention, there is provided a blood oxygenator comprising: a generally cylindrical housing having proximal and distal ends and including a blood inlet port, a blood outlet port, a heat exchange fluid inlet port, a heat exchange fluid outlet port, a gas inlet port, and a gas outlet port, the housing enclosing respective centre, intermediate and outer annular chamber extending longitudinally within the device, the chambers separated by porous wall members which permit blood to pass therethrough without substantial resistance; the centre chamber being in fluid communication with the blood inlet port and the outer chamber being in fluid communication with the blood outlet port so as to provide a blood flow path from the blood inlet port, through the centre, intermediate and outer chambers to the blood outlet port; an annular bundle of open-ended hollow heat exchange fibres disposed in the intermediate chamber and having proximal and distal ends, fibres of the heat exchange fibre bundle being arranged so as to have one open end at the proximal end of the bundle and the other open end at the distal end of the bundle, first and second sealing means at the respective proximal and distal ends of the heat exchange fibre bundle providing sealed separation of the fibre ends from the blood flow path, the open fibre ends at one end of the heat exchange fibre bundle being in fluid communication with the heat exchange fluid inlet port and the open fibre ends at the other end of the heat exchange fibre bundle being in fluid communication with the heat exchange fluid outlet port so as to provide a heat exchange fluid flow path from the heat exchange fluid inlet port through the hollow fibres of the heat exchange fibre bundle to the heat exchange fluid outlet port, an annular bundle of open ended hollow gas exchange fibres disposed in the outer chamber and having proximal and distal ends, each fibre of the gas exchange bundle being arranged so as to have one open end at the proximal end of the bundle and the other open end at the distal end of the bundle, third and fourth sealing means at the respective proximal and distal ends of the gas exchange fibre bundle providing sealed separation of the fibre ends from the blood and heat flow paths, the open fibre bundle being in fluid communication with the gas inlet port and the open fibre ends at the other end of the gas exchange fibre bundle being in fluid communication with the gas outlet port so as to provide a gas flow path from the gas inlet port through the hollow fibres of said gas exchange fibre bundle to the gas outlet port.

The invention extends, more generally to a combined blood heater oxygenator device comprising a hollow generally cylindrical core defining a longitudinally extending central chamber, an annular bundle of generally longitudinally extending heat exchange fibres disposed about the core, and a annular bundle of generally longitudinally extending gas exchange fibres disposed about the bundle of heat exchange fibres, the device providing a blood flow path extending radially outwards from the core through the heat exchange fibre bundle and then through the gas exchange fibre bundle.

The present invention thus pertains to a novel compact integrated blood heater/oxygenator in which the blood advantageously flows transversely to the axial direction of hollow heat exchange and oxygenation fibers, the device having a minimal priming volume and which is easily assembled using conventional fiber winding techniques for packing the gas exchange fibers.

The inventive blood heater oxygenator is a generally cylindrical device which is constructed so that the blood enters a central chamber extending longitudinally along the axis of the device and then moves radially through respective annular hollow heat exchange and oxygenation fiber bundles in a direction generally perpendicular to the axis of the device and generally transverse to the axial direction of the fibers toward the outer wall of the device where the temperature is adjusted and oxygenated blood is collected and passed out of the device via an exit port.

The invention may be carried into practice in various ways and some embodiments will be described by way of example to reference to the accompanying drawings, in which:-
Fig. 1 is a side perspective view of a blood heat exchanger/oxygenator of the invention.
Fig. 2 is a side plan view with parts cut away of the heat exchanger/oxygenator of Fig. 1.
Fig. 3 shows a sectional view of the heat exchanger/oxygenator of the invention taken along line 3-3 of Fig. 2.
Fig. 4 is a side view of a portion of the device as seen from line 4-4 of Fig. 3.
Fig. 5 is an enlarged perspective view of the portion of Fig. 2 indicated by the bold numeral 5.

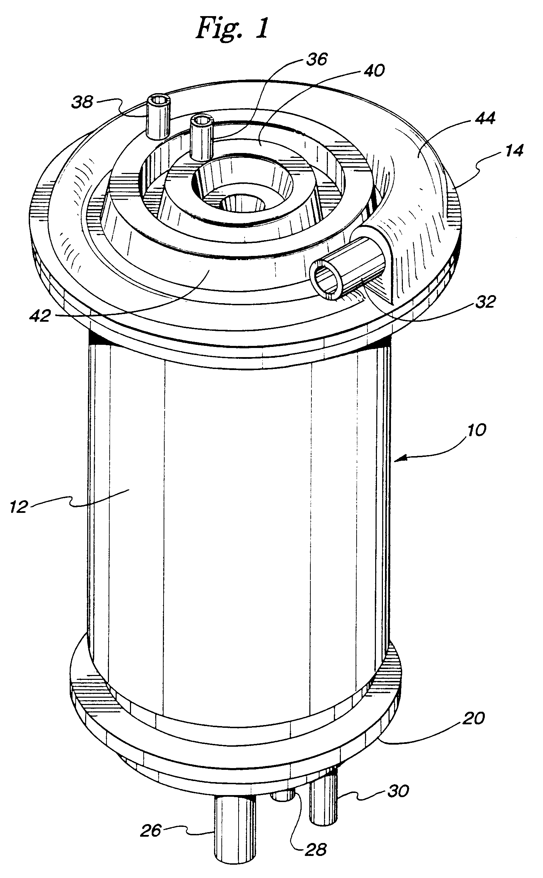

The invention is best described by reference to the preferred embodiment as illustrated in Figs. 1-5.

The preferred heat exchanger/oxygenator device of the invention is generally designated in the figures by the numeral 10. The exterior of device 10 comprises a generally cylindrical exterior wall portion 12, proximal cover member 14 and distal cover member 20. The distal cover 20 includes a central blood inlet port 26, a heating fluid outlet port 28 and a gas outlet port 30.

The proximal cap 14 includes a blood outlet port 32, a heater exchange fluid inlet port 36 and a gas inlet port 38. Raised circular portions 40 and 42 define heat exchange fluid and gas distribution manifolds, respectively, which provide fluid communication between the respective inlet ports 36 and 38 and respective hollow bundles of heat exchange and gas exchange fibers, respectively, within the device. A raised circular portion 44 defines a blood collecting manifold which, as shown in Fig. 1, increases in dimension as it approaches the exit port 32.

On the distal cover 16 there are also included raised circular portions 46 and 48 which define manifolds for collecting and directing heat exchange fluid and oxygenation gas from the fiber bundles to their respective outlet ports.

The interior of the device includes a series of annular cylindrical chambers 50, 54, 58 and 62 separated by tubular porous wall members 52, 56 and 60.

The central chamber 50 communicates with blood inlet port 26. The next outward annular chamber 54 comprises the heat exchanger portion of the device and is filled with heat exchange tubes 70 of known type which extend generally in an axial direction. Annular chamber 58 comprises the oxygenator portion of the device and is filled with tubes 74 of a gas exchange membrane material, also of known type. The gas exchange tubes 74 are also preferably oriented generally in an axial direction. Between the porous wall 60 and the inner surface of the outer wall 12 of the device is a hollow cylindrical blood collection chamber 62.

The tubular porous walls 52, 56, 60, the heat exchange tubes 70, and gas exchange tubes 74 are all potted together with a conventional potting material 76 which holds the various interior components of the device together as a unit and isolates the open ends of the tubes 70 and 74 from the blood flow path.

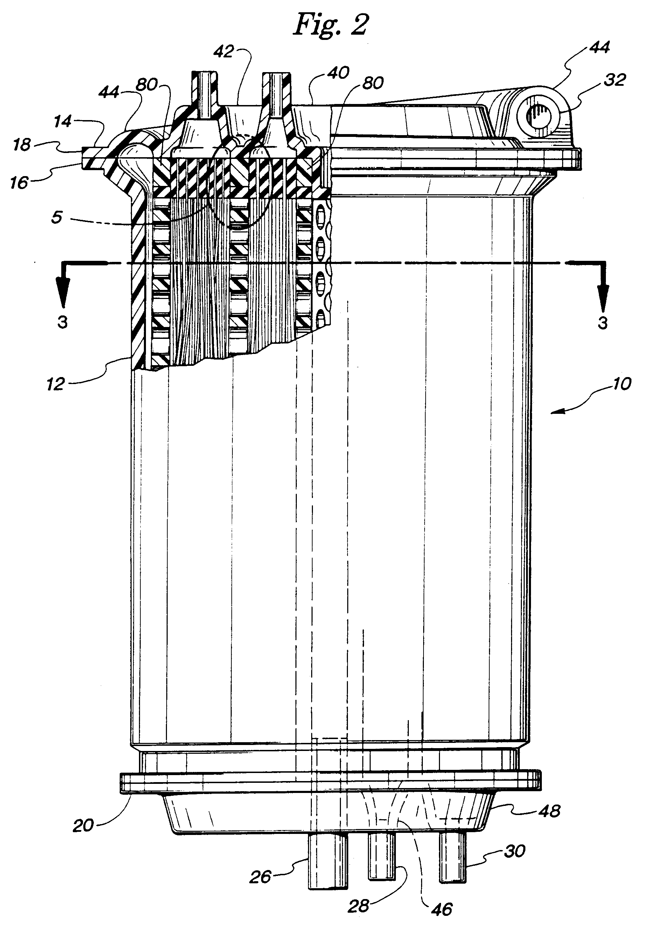

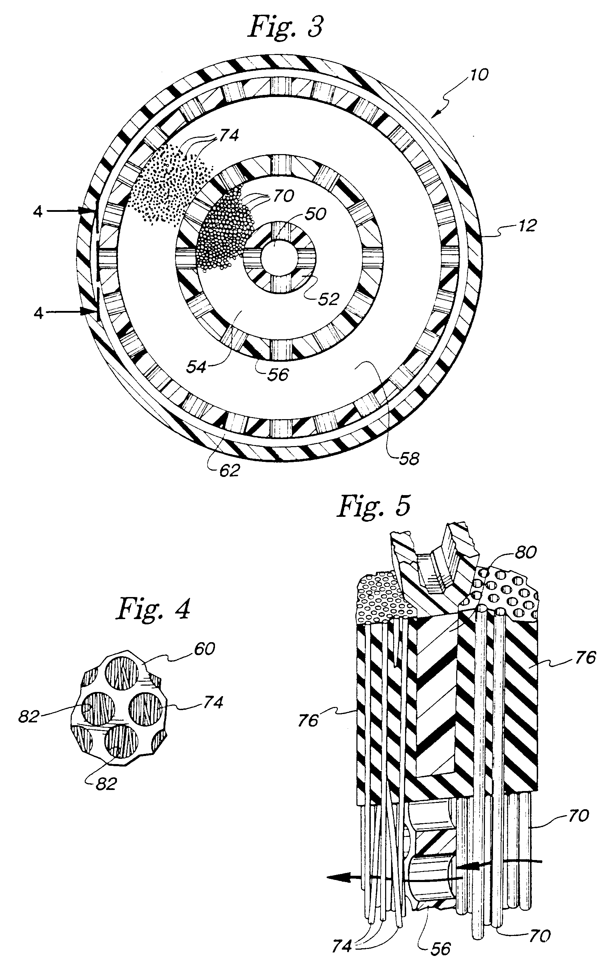

The respective bundles of heat exchange and gas exchange fibers are desirably simultaneously end potted so as to produce a unitary assembly which can be readily sheared to produce open tube ends as best shown in Fig. 5. The covers 14 and 16 are aligned so that they sealingly engage the potted assembly between the respective fiber bundles. Suitably the porous tubular wall members 52, 56 and 60 are provided with continuous non-porous end portions 80 entrained in the potting material such that when the potted assembly is sheared the end portions 80 expose continuous annular rings which provide sealing surface to engage the covers and isolate the respective gas blood and heating fluid distribution and collection manifolds, as shown in Figs. 2 and 5. Most preferably the cover assemblies are heat or sonically welded to the end portions 80 and to the ends of outer cylindrical wall 12.

The tubular porous wall members 52, 56 and 60 provide separation between the chambers while allowing blood to pass therethrough without offering substantial resistance or directional change. Any porous structure which allows the passage of blood without significant damage may be used. However, it is preferred that these wall members be constructed of a biocompatible plastic material containing a plurality of spaced orifices 82. The orifices 82 are preferably no greater than 1/2 inch (1.27 cm) and preferably 3/8 inches (0.95 cm) in diameter. Larger diameter orifices will allow the fibers to bulge into the orifices and thereby potentially create void spots in the fiber bundle therebelow. Another disadvantage in fibers bulging into the orifices is that pinching to close a fiber may occur. Smaller diameter orifices may be used, but spacing must be selected so that the total area of the orifices 82 is sufficient to assure that the respective porous tubular wall members do not themselves create significant resistance to blood flow or dead spots where blood may collect and coagulate.

Suitable gas exchange membrane material for fibers 74 may be made of polypropylene, polyethylene or other biocompatible material which provides gas exchange. The fibers are liquid impermeable. Suitable fibers for this purpose are well known and commercially available from a number of manufacturers including Mitsubishi Rayon Sale, Ltd. of Tokyo, Japan and Celanese Chemical Company of New York, New York, U.S.A.

The heat exchange tubes 70 are preferably formed from a polyurethane resin such as B.F. Goodrich Estane 58091. The tubes are much larger than the hollow fibers in the oxygenator, typically being about 0.033 inches (840 microns) in outside diameter with a wall thickness of about 0.004 inches (102 microns). In contrast, a typical oxygenator fiber has an outside diameter of about 200-450 microns and a wall thickness of less than 50 microns. The formation of heat exchanger tubes from polyurethane rather than the stainless steel, polyethylene, or polypropylene is preferred. While the efficiency of the heat exchange is an important design consideration, it is vital that there must be no leakage. The end seals where polyurethane potting compounds are used with stainless steel tubes represent potential leakage areas of the cooling fluid into the blood.

The use of polyurethane heat exchange tubes with the polyurethane end potting compounds provides a positive seal which ensures that no leakage will occur. This compatibility with the potting compound greatly increases the safety of the product.

The hollow heat exchange tubes are packed into chamber 70 such that channeling is minimized. However, performance of the heat exchanger is not greatly affected if some channeling is present. A pack density of between about 40% and 60% provides an efficient heat exchanger with an acceptable pressure drop. It is preferred to pack the polyurethane tubes at about 45% - 55% pack density which provides an efficient unit, low pressure drop and low blood priming volume. The thin walled polyurethane hollow tubes provide good heat transfer. The efficiency desired is in ensuring that all of the blood is heated or cooled as desired, not in how much heat exchange fluid is required. The temperature differential between the blood and heat exchange fluid should be low to provide better control.

In the preferred embodiment the overall size of the unit is approximately 5 inches (12.5 cm) in diameter by 7.5 inches (18.75 cm) long. The heat exchange tubes are polymeric tubes having an approximate diameter of 0.033 inches (.83 mm or 830»), and the heat exchange chamber containing approximately 2750 tubes. The gas exchange fibers suitably are microporous hollow polypropylene membrane is sufficient quantity to provide a total blood contact surface area of approximately 3.8 square meters. The device permits an outlet blood oxygen tension of 150 torr or more, tested on bovine blood with a hemoglobin concentration of 12 gram-percent; with an inlet saturation of 55% a blood flow of 6 liters per minute and an oxygen flow of 6 liters per minute. The heat exchanger provides an effectiveness level of 45% as measured by the protocol of the American Association of Medical Instrumentation (AAMI).

The heat exchange tubes are preferably cut to length and then placed into the chamber 52. Winding the tubes about central core 52 is less preferable as it tends to cause the hollow tubes to bend and may cause cracks or breaks.

The gas exchange fiber bundle is most suitably prepared by spiral winding fibers 74 around the tubular wall member 56, successive layers being angled relative to each other to produce a crisscross pattern. The crossing fiber arrangement is preferred over parallel fiber packing since it forces the blood into effective but gentle transverse, mixing without traumatizing the blood. Winding techniques for producing cylindrical bundles of hollow fibers are well known and are described in such references as U.S. 3,755,034, 3,794,468, 4,224,094, 4,336,138, 4,368,124 and 4,430,219.

The preferred angle between the fibers of successive layers is in the range of between about 10' and 30', more preferably between about 15' and 20', most preferably 18'. The fibers run in a generally axial direction, so that an axial plane bisects the angle between the successive layers of the fibers. For instance, in the most preferred embodiment, one layer will deviate from the axial direction by +9' and the next layer will deviate from the axial direction by -9'. The pack density of the gas exchange fibers 74 should be between about 45% and 60%, most preferably about 50% and 55%. When the pack density is too high the resulting resistance to blood flow reduces oxygenation efficiency. Likewise, when the pack density is too low channeling and reduced turbulent flow of the blood also reduces oxygenation efficiency. Within the preferred range oxygenation efficiency is maximized.

For potting the ends of the assembly of fiber bundles and porous wall members 52, 56 and 60, a polyurethane potting compound is preferred. Suitable potting compounds are available from Caschem, Inc. of Bayonne, New Jersey, U.S.A. A polyurethane casting system of Caschem, Inc. is described in United States Reissue Patent 31,389. After potting the hollow fibers are reopened by conventional techniques such as shearing the potting with a sharp knife so as to expose the interiors of the fibers.

After insertion of the potted and sheared assembly into cylinder 12 the cover members 14 and 20 are inserted in line so that they sealingly engage the potted assembly between the respective fiber bundles.

The covers 14 and 20, cylinder case 12 and the porous tubular wall members 52, 56 and 60 are all preferably made from nontoxic biocompatible plastic resins. Suitable resins are polycarbonate resins such as the Lexan brand resins of General Electric Company, Polymer Product Department, Pittsfield, Massachusetts. Lexan 144 grade polycarbonate resins are currently preferred.

In operation, blood entering the device through the central inlet port 26, fills chamber 50 and then passes in a direction generally perpendicular to the axis through porous wall 52, around heat exchange fibers 70, through porous wall 56, around gas exchange fibers 74, through wall 60, into collection chamber 62 and then up into the blood collecting manifold 44 in cover 14, finally exiting the device via blood exit port 32.

An advantage provided by the compact structure of the device is a reduction in priming volume which results because blood is directly passed from the heat exchange chamber 54 to the oxygenation chamber 58 without passing through intermediate collection and distribution manifolds.

Yet another advantage of the invention compared to many of the prior art devices described in the Background section, above, is the location of the heat exchange chamber upstream from the gas exchange chamber. Since gas solubility varies significantly with temperature, it is important that the blood is oxygenated at the temperature it will enter the body. If the blood is heated after it is oxygenated, the oxygenation level may exceed the gas saturation point at the higher temperature, resulting in formation of dangerous emboli. If blood is cooled after oxygenation inefficient oxygenation can result.

Compared to the rectangular devices of WO 89/0864 and PCT/US89/00146, the device of the present invention also provides a significantly less complicated device to manufacture. In particular, to obtain the desired angular and offset orientation of the gas exchange fibers in the prior art rectangular device it was necessary to employ a manufacturing technique which not only laid alternate layers in a crisscross pattern angled with respect to each other approximately 18', but also required offsetting each successive parallel layer to minimize channeling. In the cylindrical device of the invention the desired crisscrossing of successive layers can readily be performed by conventional spiral winding techniques and the increasing diameter of the winding naturally results in an offset of successive parallel layers without complex controls.

## Claims

1. A blood oxygenator (10) comprising: a generally cylindrical housing (12) having proximal and distal ends and including a blood inlet port (26), a blood outlet port (32), a heat exchange fluid inlet port (36), a heat exchange fluid outlet port (28), a gas inlet port (38), and a gas outlet port (30), the housing enclosing respective centre, intermediate and outer annular chambers (50,54,58,62) extending longitudinally within the device, the chambers separated by porous wall members (52,56,60) which permit blood to pass therethrough without substantial resistance; the centre chamber (50) being in fluid communication with the blood inlet port (26) and the outer chamber (58) being in fluid communication with the blood outlet port (32) so as to provide a blood flow path from the blood inlet port (26), through the centre, intermediate and outer chambers to the blood outlet port (32); an annular bundle of open-ended hollow heat exchange fibres (70) disposed in the intermediate chamber (54) and having proximal and distal ends, fibres of the heat exchange fibre bundle being arranged so as to have one open end at the proximal end of the bundle and the other open end at the distal end of the bundle, first and second sealing means (76) at the respective proximal and distal ends of the heat exchange fibre bundle (70) providing sealed separation of the fibre ends from the blood flow path, the open fibre ends at one end of the heat exchange fibre bundle being in fluid communication with the heat exchange fluid inlet port (36) and the open fibre ends at the other end of the heat exchange fibre bundle being in fluid communication with the heat exchange fluid outlet port (28) so as to provide a heat exchange fluid flow path from the heat exchange fluid inlet port through the hollow fibres (20) of the heat exchange fibre bundle to the heat exchange fluid outlet port (28), an annular bundle of open ended hollow gas exchange fibres (74) disposed in the outer chamber (58) and having proximal and distal ends, each fibre of the gas exchange bundle (74) being arranged so as to have one open end at the proximal end of the bundle and the other open end at the distal end of the bundle, third and fourth sealing means (76) at the respective proximal and distal ends of the gas exchange fibre bundle (74) providing sealed separation of the fibre ends from the blood and heat flow paths, the open fibre bundle (74) being in fluid communication with the gas inlet port (38) and the open fibre ends at the other end of the gas exchange fibre bundle being in fluid communication with the gas outlet port (30) so as to provide a gas flow path from the gas inlet port (38) through the hollow fibres of said gas exchange fibre bundle (74) to the gas outlet port (30).

2. A blood oxygenator as claimed in Claim 1 further comprising an annular blood collection chamber (62) between the outer chamber (58) and the inner wall of the cylindrical housing (12), the blood outlet port (32) opening into the blood collection chamber (62) and the outer chamber (58) separated from the blood collection chamber by a porous wall membrane (60) which permits blood to pass therethrough without substantial resistance.

3. A blood oxygenator as claimed in Claim 1 or Claim 2, in which the gas exchange fibres (74) are layered concentrically, the successive layers being angled relative to each other.

4. A blood oxygenator as claimed in Claim 3, in which the angle between the gas exchange fibre layers is between about 10' and 30'.

5. A blood oxygenator as claimed in Claim 4, in which the angle is about 18'.

6. A blood oxygenator as claimed in any of Claims 3 to 5, in which a plane passing through the axis of the device bisects the angle between the successive layers of gas exchange fibres (74).

7. A blood oxygenator as claimed in any preceding Claim, in which the packing density of the gas exchange fibres (74) is between about 45% and 60%.

8. A blood oxygenator as claimed in Claim 7, in which the packing density is between about 50% and 55%.

9. A blood oxygenator as claimed in any preceding Claim, in which the heat exchange fibres are polyurethane fibres.

10. A blood oxygenator as claimed in any of Claims 2 to 9, in which the fibre bundles and the said porous wall membrane are unitised by potting the ends of the fibre bundles (70,74) and the porous wall members (52,56,60) together.

11. A blood oxygenator as claimed in Claim 10, in which the end potting material (76) is a polyurethane.

12. A blood oxygenator as claimed in any of Claims 1 to 11, in which the housing comprises a cylindrical body member (12) with proximal and distal end cover members (14,16) sealingly affixed thereto, the blood, heat exchange fluid and gas exchange fluid inlet and outlet ports being located on the cover members (14,16).

13. A blood oxygenator as claimed in Claim 12, in which the proximal cover (14) includes the blood outlet port (32), the heat exchange fluid inlet port (36) and the gas inlet port (38) and the distal cover (16) include the blood inlet (26, gas outlet (30) and heat exchange outlet (28) ports thereon.

14. A blood oxygenator as claimed in Claim 13, in which the proximal cover (14) is configured to provide a circular blood conveying manifold (44) for conveying blood from the blood collection chamber (62) to the blood outlet port (32) and circular gas and heat exchange fluid distribution manifolds (42,40) providing fluid communication between the respective gas and heat exchange fluid inlet ports (38,36) and the proximal exits of the respective gas exchange and heat exchange fibres (74,70); and in which the distal cover (16) is configured to provide fluid communication between the blood inlet port (26) and the central chamber (50) and circular gas and heat exchange fluid exit manifolds (48,46) providing fluid communication between the distal ends of the gas and heat exchange fibres (74,70), respectively, and the respective gas and heat exchange fluid exit ports (30,28).

15. A combined blood heater oxygenator device comprising a hollow generally cylindrical core defining a longitudinally extending central chamber (50), an annular bundle of generally longitudinally extending heat exchange fibres (70) disposed about the core, and a annular bundle of generally longitudinally extending gas exchange fibres (74) disposed about the bundle of heat exchange fibres (70), the device providing a blood flow path extending radially outwards from the core through the heat exchange fibre bundle (70) and then through the gas exchange fibre bundle (74).

16. A device as claimed in Claim 15, in which the gas exchange fibres (74) are spirally wound in concentric layers extending generally longitudinally, the successive layers being angled relative to each other at an angle of between about 10' and 30'.

17. A device as claimed in Claim 16, in which a plane passing through the axis of the device bisects the angle between the successive layers of gas exchange fibres (74).

18. A device as claimed in Claim 17, in which the angle is about 18'.

## Patentansprüche

1. Zur Sauerstoffanreicherung in Blut dienende Einrichtung (10), welche: ein im allgemeinen zylindrisches Gehäuse (12) aufweist, das proximale und distale Enden besitzt und eine Bluteinlaßöffnung (26), eine Blutauslaßöffnung (32), eine Wärmeaustauschfluidseinlaßöffnung (36), eine Wärmeaustauschfluidauslaßöffnung (28), eine Gaseinlaßöffnung (38) sowie eine Gasauslaßöffnung (30) aufweist, wobei das Gehäuse entsprechende zentrale, dazwischenliegende und äußere ringförmige Kammern (50, 54, 58, 62) umschließt, welche sich in Längsrichtung innerhalb der Vorrichtung erstrecken, wobei die Kammern durch poröse Wandungsglieder (52, 56, 60) getrennt sind, welche dem Blut den Durchtritt durch sie ohne erheblichen Widerstand ermöglichen; wobei die zentrale Kammer (50) in Fluidkommunikation mit der Bluteinlaßöffnung (26) und die äußere Kammer (58) in Fluidkommunikation mit der Blutauslaßöffnung (32) stehen, um einen Blutfließweg von der Bluteinlaßöffnung (26) durch die zentrale, die dazwischenliegende und die äußere Kammer zur Blutauslaßöffnung (32) vorzusehen; ein ringförmiges Bündel aus hohlen, offene Enden aufweisenden Wärmeaustauschfasern (70), welche in der dazwischenliegenden Kammer (54) angeordnet sind und proximale sowie distale Enden aufweisen, wobei die Fasern des Wärmeaustauschfaserbündels derart angeordnet sind, daß sie ein offenes Ende am proximalen Ende des Bündels und das andere offene Ende am distalen Ende des Bündels aufweisen, erste und zweite Dichtungsmittel (76) an den jeweiligen proximalen und distalen Enden des Wärmeaustauschfaserbündels (70), welche für eine dichte Trennung der Faserenden vom Blutfließweg sorgen, wobei die offenen Faserenden an einem Ende des Wärmeaustauschfaserbündels in Fluidkommunikation mit der Wärmeaustauschfluideinlaßöffnung (36) und die offenen Faserenden am anderen Ende des Wärmeaustauschfaserbündels in Fluidkommunikation mit der Wärmeaustauschfluidauslaßöffnung (28) stehen, um einen Wärmeaustauschfluidfließweg von der Wärmeaustauschfluideinlaßöffnung durch die hohlen Fasern (20) des Wärmeaustauschfaserbündels zur Wärmeaustauschfluidauslaßöffnung (28) vorzusehen, ein ringförmiges Bündel hohler, offene Enden aufweisender Gasaustauschfasern (74), welche in der äußeren Kammer (58) angeordnet sind und proximale sowie distale Enden aufweisen, wobei jede Faser des Gasaustauschbündels (74) derart angeordnet ist, daß sie ein offenes Ende am proximalen Ende des Bündels und das andere offene Ende am distalen Ende des Bündels aufweist, dritte und vierte Dichtungsmittel (76) an den jeweiligen proximalen und distalen Enden des Gasaustauschfaserbündels (74), welche für eine dichte Trennung der Faserenden vom Blut und vom Wärmefließweg sorgen, wobei das offene Faserbündel (74) in Fluidkommunikation mit der Gaseinlaßöffnung (38) und die offenen Faserenden am anderen Ende des Gasaustauschfaserbündels in Fluidkommunikation mit der Gasauslaßöffnung (30) stehen, um einen Gasfließweg von der Wärmeeinlaßöffnung (28) durch die hohlen Fasern des Gasaustauschfaserbündels (74) zur Gasauslaßöffnung (30) vorzusehen.

2. Zur Sauerstoffanreicherung in Blut dienende Einrichtung nach Anspruch 1, die weiters eine ringförmige Blutsammelkammer (62) zwischen der äußeren Kammer (58) und der Innenwand des zylindrischen Gehäuses (12) aufweist, wobei die Blutauslaßöffnung (32) in die Blutsammelkammer (62) mündet und die äußere Kammer (58) von der Blutsammelkammer durch eine poröse Wandungsmembran (60) getrennt wird, welche dem Blut den Durchtritt durch sie ohne erheblichen Widerstand ermöglicht.

3. Zur Sauerstoffanreicherung in Blut dienende Einrichtung nach Anspruch 1 oder Anspruch 2, bei welcher die Gasaustauschfasern (74) konzentrisch geschichtet sind, wobei die aufeinanderfolgenden Schichten relativ zueinander in einem Winkel angeordnet sind.

4. Zur Sauerstoffanreicherung in Blut dienende Einrichtung nach Anspruch 3, bei welcher der Winkel zwischen den Schichten der Gasaustauschfasern zwischen ungefähr 10' und 30' beträgt.

5. Zur Sauerstoffanreicherung in Blut dienende Einrichtung nach Anspruch 4, bei welcher der Winkel ungefähr 18' beträgt.

6. Zur Sauerstoffanreicherung in Blut dienende Einrichtung nach einem der Ansprüche 3 bis 5, bei welcher eine Ebene, die durch die Achse der Vorrichtung verläuft, den Winkel zwischen den aufeinanderfolgenden Schichten aus Gasaustauschfasern (74) halbiert.

7. Zur Sauerstoffanreicherung in Blut dienende Einrichtung nach einem der vorangehenden Ansprüche, bei welcher die Packdichte der Gasaustauschfasern (74) zwischen ungefähr 45% und 60% beträgt.

8. Zur Sauerstoffanreichung in Blut dienende Einrichtung nach Anspruch 7, bei welcher die Packdichte zwischen ungefähr 50% und 55% beträgt.

9. Zur Sauerstoffanreicherung in Blut dienende Einrichtung nach einem der vorangehenden Ansprüche, bei welcher die Wärmeaustauschfasern Polyurethanfasern sind.

10. Zur Sauerstoffanreicherung in Blut dienende Einrichtung nach einem der Ansprüche 2 bis 9, bei welcher die Faserbündel und die poröse Wandungsmembran durch gemeinsames Vergießen der Enden der Faserbündel (70, 74) und der porösen Wandungsglieder (52, 56, 60) zu einer Einheit vereinigt sind.

11. Zur Sauerstoffanreicherung in Blut dienende Einrichtung nach Anspruch 10, bei welcher das Material zum Vergießen der Enden (76) ein Polyurethan ist.

12. Zur Sauerstoffanreicherung in Blut dienende Einrichtung nach einem der Ansprüche 1 bis 11, bei welcher das Gehäuse einen zylindrischen Körper (12) mit proximalen und distalen Endabdeckungen (14, 16) umfaßt, welche abdichtend daran befestigt sind, wobei die Blut-, Wärmeaustauschfluid- und Gasaustauschfluideinlaß- und -auslaßöffnungen auf den Abdeckungen (14, 16) angeordnet sind.

13. Zur Sauerstoffanreicherung in Blut dienende Einrichtung nach Anspruch 12, bei welcher die proximale Abdeckung (14) die Blutauslaßöffnung (32), die Wärmeaustauschfluideinlaßöffnung (36) und die Gaseinlaßöffnung (38) enthält und die distale Abdeckung (16) die Bluteinlaßöffnung (26), die Gasauslaßöffnung (30) und die Wärmeaustauschauslaßöffnung (28) enthält.

14. Zur Sauerstoffanreicherung in Blut dienende Einrichtung nach Anspruch 13, bei welcher die proximale Abdeckung (14) derart konfiguriert ist, daß sie einen kreisförmigen Bluttransportverteiler (44) zum Transportieren von Blut aus der Blutsammelkammer (62) zur Blutauslaßöffnung (32) sowie kreisförmige Gas- und Wärmeaustauschfluidverteiler (42, 40) vorsieht, welche für eine Fluidkommunikation zwischen den jeweiligen Gas- und Wärmeaustauschfluideinlaßöffnungen (38, 36) und den proximalen Ausgängen der jeweiligen Gasaustausch- und Wärmeaustauschfasern (74, 70) sorgen; und bei welchem die distale Abdeckung (16) derart konfiguriert ist, daß sie eine Fluidkommunikation zwischen der Bluteinlaßöffnung (26) und der zentralen Kammer (50) und kreisförmige Gas- und Wärmeaustauschfluidausgangsverteiler (48, 46) vorsieht, welche für Fluidkommunikation zwischen den distalen Enden der Gas- bzw. Wärmeaustauschfasern (74, 70) und den jeweiligen Gas- und Wärmeaustauschfluidausgangsöffnungen (30, 28) sorgen.

15. Kombinierte Blutwärme- und Sauerstoffanreicherungsvorrichtung, welche einen hohlen, im allgemeinen zylindrischen Kern, der eine sich in Längsrichtung erstreckende zentrale Kammer (50) begrenzt, ein ringförmiges Bündel aus sich im allgemeinen in Längsrichtung erstreckenden, um den Kern herum angeordneten Wärmeaustauschfasern (70) und ein ringförmiges Bündel aus sich im allgemeinen in Längsrichtung erstreckenden, um das Bündel aus Wärmeaustauschfasern (70) herum angeordneten Gasaustauschfasern (74) enthält, wobei die Vorrichtung einen Blutfließweg vorsieht, welcher sich radial vom Kern nach außen durch das Wärmeaustauschfaserbündel (70) und hernach durch das Gasaustauschfaserbündel (74) erstreckt.

16. Vorrichtung nach Anspruch 15, bei welcher die Gasaustauschfasern (74) in konzentrischen, sich in Längsrichtung erstreckenden Schichten spiralförmig gewunden sind, wobei die aufeinanderfolgenden Schichten relativ zueinander in einem Winkel von ungefähr 10' bis ungefähr 30' angeordnet sind.

17. Vorrichtung nach Anspruch 16, bei welcher eine Ebene, die durch die Achse der Vorrichtung verläuft, den Winkel zwischen den aufeinanderfolgenden Schichten aus Gasaustauschfasern (74) halbiert.

18. Vorrichtung nach Anspruch 17, bei welcher der Winkel ungefähr 18' beträgt.

## Revendications

1. Oxygénateur de sang (10) comprenant : un boîtier généralement cylindrique (12) qui présente une extrémité proximale et une extrémité distale et qui comporte un orifice d'entrée du sang (26), un orifice de sortie du sang (32), un orifice d'entrée du fluide d'échange de chaleur (36), un orifice de sortie du fluide d'échange de chaleur (28), un orifice d'entrée du gaz (38) et un orifice de sortie du gaz (30), le boitier renfermant des chambres respectivement centrale, intermédiaires et annulaire externe (50, 54, 58, 62) qui s'étendent longitudinalement dans l'appareil, les chambres étant séparées par des parois poreuses (52, 56, 60) qui laissent le sang les traverser sans présenter de résistance substantielle; la chambre centrale (50) communiquant avec l'orifice d'entrée du sang (26) et la chambre externe (58) communiquant avec l'orifice de sortie du sang (32) de manière à fournir un chemin d'écoulement du sang allant de l'orifice d'entrée du sang (26), par les chambres centrale, intermédiaires et externe, jusqu'à l'orifice de sortie du sang (32); un faisceau annulaire (70) de fibres d'échange de chaleur, creuses, ouvertes à leurs extrémités disposées dans la chambre intermédiaire (54) et présentant une extrémité proximale et une extrémité distale, les fibres du faisceau de fibres d'échange de chaleur étant disposées de manière à présenter une extrémité ouverte à l'extrémité proximale du faisceau et l'autre extrémité ouverte à l'extrémité distale du faisceau, un premier et deuxième moyens d'étanchéité (76) aux extrémités respectives, proximale et distale, du faisceau (70) de fibres d'échange de chaleur fournissant une séparation étanche des extrémités des fibres à l'égard du chemin d'écoulement du sang, les extrémités ouvertes des fibres à une extrémité du faisceau de fibres d'échange de chaleur communiquant avec l'orifice d'entrée du fluide d'échange de chaleur (36) et les extrémités ouvertes des fibres à l'autre extrémité du faisceau de fibres d'échange de chaleur communiquant avec l'orifice de sortie du fluide d'échange de chaleur (28) de manière à fournir un chemin d'écoulement du fluide d'échange de chaleur allant de l'orifice d'entrée du fluide d'échange de chaleur, par les fibres creuses (20) du faisceau de fibres d'échange de chaleur, jusqu'à l'orifice de sortie du fluide d'échange de chaleur (28), un faisceau annulaire (74) de fibres d'échange de gaz, creuses, ouvertes à leurs extrémités, disposées dans la chambre externe (58) et présentant une extrémité proximale et une extrémité distale, chaque fibre du faisceau (74) d'échange de gaz étant disposée de manière à présenter une extrémité ouverte à l'extrémité proximale du faisceau et l'autre extrémité ouverte à l'extrémité distale du faisceau, un troisième et quatrième moyens d'étanchéité (76) aux extrémités respectives, proximale et distale, du faisceau (74) de fibres d'échange de gaz fournissant une séparation étanche des extrémités des fibres à l'égard des chemins d'écoulement du sang et de la chaleur, le faisceau (74) de fibres ouvertes communiquant avec l'orifice d'entrée du gaz (38) et les extrémités ouvertes des fibres à l'autre extrémité du faisceau de fibres d'échange de gaz communiquant avec l'orifice de sortie du gaz (30) de manière à fournir un chemin d'écoulement du gaz allant de l'orifice d'entrée de la chaleur (28), par les fibres creuses dudit faisceau (74) de fibres d'échange de gaz, jusqu'à l'orifice de sortie du gaz (30).

2. Oxygénateur de sang suivant la revendication 1, comprenant, en outre, une chambre annulaire de collecte de sang (62) entre la chambre externe (58) et la paroi interne du boîtier cylindrique (12), l'orifice de sortie du sang (32) débouchant dans la chambre de collecte de sang (62) et dans la chambre externe (58) séparée de la chambre de collecte de sang par une membrane poreuse de paroi (60) qui laisse le sang la traverser sans présenter de résistance substantielle.

3. Oxygénateur de sang suivant la revendication 1 ou la revendication 2, dans lequel les fibres (74) d'échange de gaz sont disposées en couches concentriques, les couches successives formant un angle les unes par rapport aux autres.

4. Oxygénateur de sang suivant la revendication 3, dans lequel l'angle entre les couches de fibres d'échange de gaz est compris entre environ 10' et 30'.

5. Oxygénateur de sang suivant la revendication 4, dans lequel l'angle est d'environ 18'.

6. Oxygénateur de sang suivant l'une quelconque des revendications 3 à 5, dans lequel un plan qui passe par l'axe de l'appareil divise l'angle entre les couches successives de fibres d'échange de gaz (74) en deux angles égaux.

7. Oxygénateur de sang suivant l'une quelconque des revendications précédentes, dans lequel la densité de tassement des fibres d'échange de gaz (74) est comprise entre environ 45% et 60%.

8. Oxygénateur de sang suivant la revendication 7, dans lequel la densité de tassement des fibres est comprise entre environ 50% et 55%.

9. Oxygénateur de sang suivant l'une quelconque des revendications précédentes, dans lequel les fibres d'échange de chaleur sont des fibres de polyuréthanne.

10. Oxygénateur de sang suivant l'une quelconque des revendications 2 à 9, dans lequel les faisceaux de fibres et ladite membrane poreuse de paroi sont unifiés en enrobant ensemble les extrémités des faisceaux de fibres (70, 74) et les parois poreuses (52, 56, 60).

11. Oxygénateur de sang suivant la revendication 10, dans lequel la matière d'enrobage d'extrémité (76) est un polyuréthanne.

12. Oxygénateur de sang suivant l'une quelconque des revendications 1 à 11, dans lequel le boîtier comprend un corps cylindrique (12) muni de couvercles (14, 16) pour l'extrémité proximale et l'extrémité distale, qui sont fixés de manière étanche à ce corps, les orifices d'entrée et de sortie du sang, du fluide d'échange de chaleur et du fluide d'échange de gaz étant situés sur les organes de couverture (14, 16).

13. Oxygénateur de sang suivant la revendication 12, dans lequel le couvercle proximal (14) comporte l'orifice de sortie du sang (32), l'orifice d'entrée du fluide d'échange de chaleur (36) et l'orifice d'entrée du gaz (38) et le couvercle distal (16) comporte les orifices d'entrée du sang (26), de sortie du gaz (30) et de sortie du fluide d'échange de chaleur (28).

14. Oxygénateur de sang suivant la revendication 13, dans lequel le couvercle proximal (14) est configuré de manière à fournir une tubulure (44) circulaire d'acheminement du sang destinée à acheminer le sang depuis la chambre de collecte du sang (62) jusqu'à l'orifice de sortie du sang (32) et des tubulures (42, 40) circulaires de distribution des fluides d'échange de gaz et d'échange de chaleur fournissant une communication entre les orifices (38, 36) respectifs d'entrée des fluides d'échange de gaz et d'échange de chaleur et les sorties proximales des fibres (74, 70) respectives d'échange de gaz et d'échange de chaleur; et dans lequel le couvercle distal (16) est configuré de manière à fournir une communication entre l'orifice d'entrée du sang (26) et la chambre centrale (50) et les tubulures (48, 46) circulaires de sortie des fluides d'échange de gaz et d'échange de chaleur fournissant une communication entre les extrémités distales des fibres (74, 70) d'échange de gaz et d'échange de chaleur, respectivement, et les orifices (30, 28) respectifs de sortie des fluides d'échange de gaz et d'échange de chaleur.

15. Appareil combiné pour chauffer et oxygéner le sang comprenant un noyau creux généralement cylindrique qui définit une chambre centrale (50) qui s'étend longitudinalement, un faisceau annulaire de fibres (70) d'échange de chaleur s'étendant dans l'ensemble longitudinalement disposées autour du noyau, et un faisceau annulaire de fibres (74) d'échange de gaz s'étendant dans l'ensemble longitudinalement, disposées autour du faisceau de fibres (70) d'échange de chaleur, l'appareil fournissant un chemin d'écoulement pour le sang qui s'étend radialement vers l'extérieur depuis le noyau à travers le faisceau (70) de fibres d'échange de chaleur et ensuite, à travers le faisceau (74) de fibres d'échange de gaz.

16. Appareil suivant la revendication 15, dans lequel les fibres (74) d'échange de gaz sont enroulées en hélice dans des couches concentriques qui s'étendent dans l'ensemble longitudinalement, les couches successives formant l'une par rapport à l'autre un angle compris entre environ 10' et 30'.

17. Appareil suivant la revendication 16, dans lequel un plan qui passe par l'axe de l'appareil divise l'angle entre les couches successives de fibres (74) d'échange de gaz en deux angles égaux.

18. Appareil suivant la revendication 17, dans lequel l'angle est d'environ 18'.
